# EUROPEAN PATENT SPECIFICATION

(11) **EP 2 841 949 B1**
(45) Date of publication and mention of the grant of the patent: **22.11.2017**
(21) Application number: 13717432.2
(22) Date of filing: 12.04.2013
(51) Int. Cl.: G01N 33/68

(54) **BIOMARKERS FOR THE THERAPY STRATIFICATION OF SYNCOPE**
BIOMARKER ZUR THERAPEUTISCHEN SCHICHTUNG VON SYNKOPEN
BIOMARQUEURS POUR LA STRATIFICATION THÉRAPEUTIQUE D'UNE SYNCOPE

(30) Priority: 26.04.2012 EP 12002935
(43) Date of publication of application: 04.03.2015
(73) Proprietor: B.R.A.H.M.S GmbH, 16761 Hennigsdorf (DE)
(72) Inventor: STRUCK, Joachim, 13465 Berlin (DE); MELANDER, Olle, S-205 02 Malmö (SE); FEDOROWSKI, Artur, S-205 02 Malmö (SE)
(74) Representative: Tomerius, Isabel
(86) International application number: PCT/EP2013/001081
(87) International publication number: WO 2013/159872

(56) References cited:
- WO-A1-2010/049178
- WO-A1-2010/128071
- H. KAUFMANN ET AL: "Plasma endothelin during upright tilt: relevance for orthostatic hypotension?", THE LANCET, vol. 338, no. 8782-8783, 1 December 1991 (1991-12-01), pages 1542-1545, XP055035855, ISSN: 0140-6736, DOI: 10.1016/0140-6736(91)92370-H
- MARY MEHTA ET AL: "Usefulness of Endothelin-1 as a Predictor of Response to Head-Up Tilt-Table Testing in Children With Syncope", THE AMERICAN JOURNAL OF CARDIOLOGY, vol. 76, no. 1-2, 1 July 1995 (1995-07-01) , pages 86-88, XP055066607, ISSN: 0002-9149, DOI: 10.1016/S0002-9149(99)80810-8
- MAGERKURTH C ET AL: "Permanent increase in endothelin serum levels in vasovagal syncope", CLINICAL AUTONOMIC RESEARCH ; AN INTERNATIONAL JOURNAL FOR FAST COMMUNICATIONS OF RESEARCH AND TREATMENT RELATED TO AUTONOMIC FUNCTION AND DYSFUNCTION, STEINKOPFF-VERLAG, DA, vol. 15, no. 4, 1 August 2005 (2005-08-01) , pages 299-301, XP019383689, ISSN: 1619-1560, DOI: 10.1007/S10286-005-0291-6
- MICHEL WHITE ET AL: "Impaired Endothelin-1 Release in Tilt-Induced Syncope", THE AMERICAN JOURNAL OF CARDIOLOGY, vol. 81, no. 4, 1 February 1998 (1998-02-01), pages 460-464, XP055066570, ISSN: 0002-9149, DOI: 10.1016/S0002-9149(97)00938-7
- A A Gasiorowska ET AL: "Hemodynamic and neuroendocrine predictors of lower body negative pressure (LBNP) intolerance in healthy young men.", J Physiol Pharmacol 56(2):179-93 (2005) PMID 15985701, 1 January 2005 (2005-01-01), pages 179-193, XP055035822, Retrieved from the Internet: URL:http://www.jpp.krakow.pl/journal/archi ve/06_05/pdf/179_06_05_article.pdf [retrieved on 2012-08-20]
- GAJEK JACEK ET AL: "[Adrenomedullin--the link between the sympathetic nervous system activation and peripheral vasodilatation in some patients with vasovagal syncope].", POLSKI MERKURIUSZ LEKARSKI : ORGAN POLSKIEGO TOWARZYSTWA LEKARSKIEGO SEP 2004 LNKD- PUBMED:15628055, vol. 17, no. 99, September 2004 (2004-09), pages 267-270, XP009162108, ISSN: 1426-9686
- DAVID L JARDINE ET AL: "Neurohormonal Response to Head-Up Tilt and Its Role in Vasovagal Syncope", THE AMERICAN JOURNAL OF CARDIOLOGY, vol. 79, no. 9, 1 May 1997 (1997-05-01), pages 1302-1306, XP055035844, ISSN: 0002-9149, DOI: 10.1016/S0002-9149(9X)00084-9
- A. FEDOROWSKI ET AL: "Novel cardiovascular biomarkers in unexplained syncopal attacks: the SYSTEMA cohort", JOURNAL OF INTERNAL MEDICINE, vol. 273, no. 4, 1 April 2013 (2013-04-01) , pages 359-367, XP055066553, ISSN: 0954-6820, DOI: 10.1111/joim.12043

## Description

### Field of the invention

The present invention is in the field of clinical diagnostics. Particularly the present invention relates to the therapy stratification of syncope.

### Background of the invention

Evaluation of transient loss of consciousness (TLOC) in the absence of obvious and definite etiology is a complex issue. TLOC is divided into traumatic and non-traumatic forms and non-traumatic TLOC is classified into epileptic seizures, psychogenic pseudosyncope, rare miscellaneous causes and syncope. For the majority of cases with uncertain diagnosis the underlying cause is a temporary circulatory impairment leading to brain hypoperfusion, traditionally classified as a syncopal episode (Blanc et al. 2002. Eur Heart J. 23:815-820*;* Bartoletti et al. 2006. Eur Heart J 27:83-88)*.*

Syncope is a TLOC characterized by rapid onset, short duration, and spontaneous complete recovery that often occurs without warning. In particular, syncope can be divided into neurally-mediated reflex syncope (NMS), orthostatic hypotension (OH) and cardiac syncope (arrhythmic or structural), although less frequent states may occur (Moya et al. 2009. Heart J. 30:2631-2671).

NMS is usually classified based on the efferent pathway most involved, i.e. sympathetic or parasympathetic: if hypotension due to a loss of upright vasoconstrictor tone predominates, the term vasodepressor type syncope is used; if bradycardia or asystole predominates, term cardioinhibitory syncope is used; and a mixed form is diagnosed if both mechanisms are present. Moreover, NMS may also be classified based on its trigger (i.e. the afferent pathway) into vasovagal syncope (WS), situational syncope (NMS associated with some specific circumstances), carotid sinus hypersensitivity (CSH) and atypical form (in which NMS occurs with uncertain or even apparently absent trigger).

In contrast to NMS, in orthostatic hypotension sympathetic efferent activity is chronically impaired so that vasoconstriction is deficient. Upon standing, blood pressure (BP) falls and syncope or pre-syncope occurs. OH is defined as an abnormal decrease in BP upon standing. Strictly from a pathophysiological point of view there is no overlap between NMS and OH, but the clinical manifestations of the two conditions frequently overlap, sometimes making differential diagnosis difficult (Moya et al. 2009. Heart J. 30:2631-2671)*.* OH can be divided into classical OH (defined as a decrease in systolic BP ≥ 20 mmHg and in diastolic BP ≥ 10 mmHg within 3 min of standing (J Neurol Sci 1996; 144: 218-219)), initial OH (characterized by a BP decrease immediately on standing of > 40 mmHg and a spontaneously and rapid return of BP to normal resulting in a short period of hypotension and symptoms (Wieling et al. 2007. Clin Sci (Lond) 112:157-165)), delayed (progressive) OH (characterized by a slow progressive decrease in systolic BP on assuming erect posture (Gibbons and Freeman 2006. Neurology 67:28-32)), and postural orthostatic tachycardia syndrome (POTS) (characterized by very marked heart rate increases of > 30 beats per min or to > 120 beats per min and instability of BP (Grubb et al. 1997. Pacing Clin Electrophysiol 20: 2205-2212)).

Cardiac syncope can be caused by arrhythmia (bradycardia, tachycardia) or structural disease (e.g. cardiac valvular disease, acute myocardial infarction/ ischemia, congenital anomalies of coronary arteries, pulmonary hypertension and others).

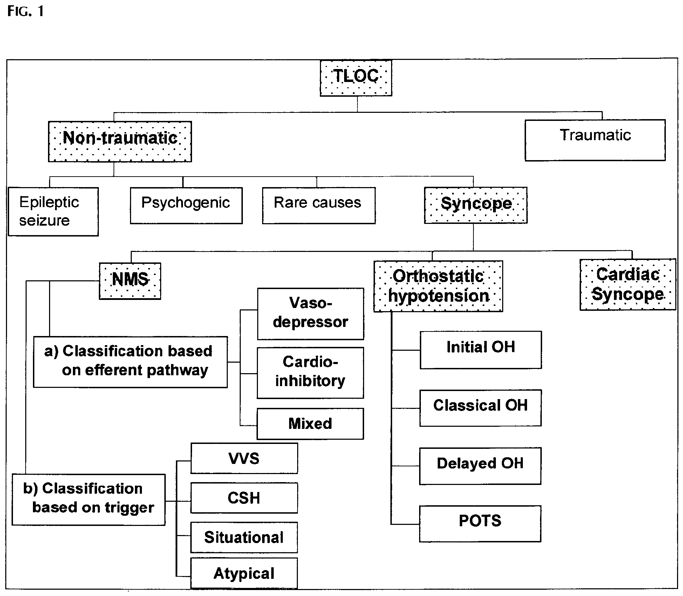

An overview for the classification of syncope is shown in Fig. 1.

According to current syncope guidelines, two main lines of investigation are recommended, an advanced cardiac assessment based on long-term ECG monitoring and autonomic tests, where head-up tilt test (HUTT) has a central role (Moya et al. 2009. Heart J. 30:2631-2671)*.* The choice of the line is often dependant on patient's history and syncope scenario.

ECG monitoring is a procedure for diagnosing intermittent brady- and tachyarrhythmias. The gold standard for the diagnosis of syncope is when a correlation between the symptoms and a documented arrhythmia is recorded. As a general rule, ECG monitoring is indicated only when there is a high pre-test probability of identifying an arrhythmia associated with syncope.

In autonomic tests an orthostatic challenge (changing from supine to upright position) produces a displacement of blood from thorax to the lower limbs that leads to a decrease in venous return and cardiac output. In the absence of compensatory mechanisms, a fall in BP may lead to syncope. Currently, there are two different methods for assessing the response to change in posture from supine to upright: the active standing test, in which patients arise actively from supine to erect, and the other is head-up tilt at 60 or 70°. The active standing test is used to diagnose different types of orthostatic intolerance using a sphygmomanometer. Tilt-testing enables the reproduction of a neurally-mediated reflex in laboratory settings. Blood pooling and decrease in venous return due to orthostatic stress and immobilization trigger the reflex. However, the diagnostic process is time-consuming and not always successful (Croci et al. 2002. Europace 4:351-355).

Biomarkers are used for diagnosis, prognosis, monitoring and therapy follow-up or stratification of different pathologies (as in applicant's other applications WO 2010/049178 A1 and WO 2010/128071 A1). Circulating levels of vasoactive hormones have been studied in patients with different types of syncope with heterogenous results. In patients with VVS, no difference at baseline (before HUT test) was found for Arginine-Vasopressin (AVP) (Kaufmann et al. 1991. Lancet 338:8782-8783*;* Kaufmann et al. 1992. Neurology 42:590-593*;* Jardine et al. 1997. Am J Cardiol 79:1302-1306*;* Roul et al. 1999. Pacing Clin Electrophysiol 22:1020-1030*;* Theopistou et al. 2001. Am J Cardiol. 88:376-381)*.* It was shown that the increase of mature AVP was more pronounced in the group with a positive HUT test result (classified as patients with VVS) compared to the negative HUT test group (Jardine et al. 1997. Am J Cardiol 79:1302-1306*;* Roul et al. 1999. Pacing Clin Electrophysiol 22:1020-1030*;* Kaufmann et al. 1991. Lancet 338:8782-8783*;* Kaufmann et al. 1992. Neurology 42:590-593*;* Theopistou et al. 2001. Am J Cardiol. 88:376-381)*.* Moreover, levels of copeptin, a stable fragment of the proAVP precursor also known as C-terminal proAVP (CT-proAVP), did not differ between patients with cardioinhibitory and vasodepressor reflex syncope (Holmegard et al. 2012. Scand J Clin Lab Invest*, in press*). Similarly, no difference was found in patients with WS for atrial or brain natriuretic peptides (ANP or BNP) (Jardine et al. 1997. Am J Cardiol 79:1302-1306*;* Roul et al. 1999. Pacing Clin Electrophysiol 22:1020-1030*;* Magerkurth et al. 2005. Clin Autonom Res 15:299-301). On the contrary, Holmegard et al. reported higher levels of mid-regional pro-atrial natriuretic peptide (MR-proANP), a stable fragment of the proANP precursor, in patients with cardioinhibitory syncope when compared to patients with vasodepressor reflex syncope, whereas levels of BNP did not differ between the groups (Holmegard et al. 2012. Scand J Clin Lab Invest, in press)*.* Similarly, Jardine et al. demonstrated a decrease of ANP during tilt in normal subjects and VVS, which was more pronounced in the normal population (Jardine et al. 1997. Am J Cardiol 79:1302-130)*.* In contrast to these results, Freitas et al. reported that supine levels of BNP are distinctly lower among patients with neurally mediated syncope as compared to patients with neurogenic OH, whereas levels of ANP where unchanged (Freitas et al 2007. Int J Cardiol 116:242-248)*.* Moreover, the difference (Δ tilt/ basal) of both ANP and BNP was not different between patients with neurally mediated syncope as compared to patients with neurogenic OH. Gajek et al. reported that there was no difference in baseline adrenomedullin concentrations in patients with cardioinhibitory reflex between those with spontaneous syncope and patients with drug-induced syncope (Gajek et al. 2004. Pol Merkur Lekarski 17: 267-270).

No difference in baseline levels of AVP was found in patients with CSH when compared to healthy controls or patients with unexplained syncope, whereas AVP in patients with CSH and patients with unexplained syncope showed a significant increase during tilt which was not evident in healthy subjects (Kenny et al. 1987. Cardiovasc Res 21:545-550).

In healthy volunteers with no history of VVS but some orthostatic intolerance, serum endothelin levels were significantly higher at baseline in subjects who experienced a syncope during HUTT compared to subjects without syncope (Magerkurth et al. 2005. Clin Autonom Res 15:299-301)*.* Furthermore, the authors stated that these results point to a permanently augmented endothelin release in subjects with a tendency for vasovagal syncope and that future studies must investigate whether endothelin serum levels can increase the specificity and sensitivity to diagnose vasovagal syncope. ET-1 increased during HUT test in both, patients with VVS and normal subjects, whereas this increase was more pronounced in patients with VVS (Kaufmann et al. 1991. Lancet 338:8782-8783). In contrast, Mehta et al. reported that baseline endothelin levels were significantly higher in patients with a history of syncope and a negative HUTT result when compared to patients with a positive HUTT result (Mehta et al. 1995. Am J Cardiol 76:86-88). However, there was no difference between the syncope subtypes cardioinhibitory, vasodepressor and mixed type and no difference between the timed samples. Also, impaired endothelin-1 release was reported in tilt-induced syncope (White et al. 1998. Am J Cardiol 81: 460-464).

Adrenomedullin (ADM) was studied in healthy volunteers and subjects were divided into high (no syncope) and low tolerance (syncopal symptoms) of orthostatic challenge according to the lower body negative pressure test (LBNP) (Gasiorowska et al. 2005. J Physiol Pharmacol 56:179-193). Baseline values of ADM were significantly higher in low tolerance subjects compared to subjects with high tolerance for orthostatic challenge whereas ADM increased similarly in both groups during the test.

However, for all these studies it is important to note that before the ESC guidelines were published in 2009 (Moya et al. 2009. Heart J. 30:2631-2671)*,* no gold standard diagnosis existed for patients with syncope and consequently no consensus regarding the different forms of orthostatic instability and their relations with syncopal attacks was available. Moreover the above described biomarker studies were performed on small selected patient series, mostly compared to healthy individuals and did not encompass the whole spectrum of underlying mechanisms. Therefore a diagnosis of a specific type of syncope was challenging and not much reliable.

Moreover, a reliable diagnosis is an important prerequisite for the selection of an appropriate therapy. The goal of therapy is primarily the prevention of recurrence and associated injuries, and improvement in quality of life. Therapy options for neutrally mediated syncope are for example physical counterpressure manoeuvres (e.g. isometric PCMs of the legs or arms like leg crossing or hand grip and arm tensing) and tilt training as well as pharmacological therapy (e.g. midodrine, an α-antagonist) and cardiac pacing. It has been reported by Brignole, that active pacemaker therapy significantly reduce syncope recurrence in patients with severe neurally mediated syncope, especially in CSH with cardioinhibition (oral presentation, American College of Cardiology. Chicago USA, 24-27 of March 2012). Actually, pacing is contraindicated in the absence of severe cardioinhibition. Therefore an accurate diagnosis and assessment of the severity of syncope is mandatory.

With regard to the prognosis (i.e. risk stratification) associated with syncope, two important elements should be considered: (i) risk of death and life-threatening events; and (ii) risk of recurrence of syncope and physical injury.

Therefore an object of the present invention is the provision of a method which allows the diagnosis and/or prognosis and/or assessment and/or therapy stratification of syncope.

### Summary of the invention

Subject of the invention is a method for the therapy stratification of syncope in a patient, the method comprising:
- determining the level of CT-proET-1 in a sample of a bodily fluid of said patient,
- correlating said level of CT-proET-1 with the therapy stratification of syncope, wherein the patients are stratified according to their need of a cardiac pacemaker by determining the level of CT-proET-1, wherein a level above a predetermined threshold level is indicative of patients suffering from orthostatic hypotension who do not need a cardiac pacemaker, and wherein a level below a predetermined threshold level is indicative of patients suffering from cardioinhibitory reflex who are in need of a cardiac pacemaker.

Preferred method variants are described in the dependent claims.

### Description of drawings

Fig. 1: Classification of syncope subtypes
Fig. 2: Box-and-whisker plot of MR-proANP values for the diagnosis of syncope (not claimed per se)
Fig. 3: Box-and-whisker plot of MR-proADM values for the diagnosis of syncope (not claimed per se)
Fig. 4: Box-and-whisker plot of CT-proET-1 values for the diagnosis of syncope
Fig. 5: Box-and-whisker plot of CT-proAVP values for the diagnosis of syncope (not claimed per se)
Fig. 6: Box-and-whisker plot of PCT values for the diagnosis of syncope (not claimed per se)
Fig. 7: Linear relationship between MR-proANP and VVS (not claimed per se)
Fig. 8: Linear relationship between CT-proET-1 and VVS
Fig. 9: Linear relationship between CT-proET-1 and OH
Fig. 10: Linear relationship and threshold-like effects between MR-proANP and CSH (not claimed per se)
Fig. 11: Linear relationship between CT-proET-1 and cardioinhibitory reflex
Fig. 12: Threshold effect between CT-proAVP and initial OH (not claimed per se)

### Detailed description of the invention

Subject of the invention is a method for the therapy stratification of syncope in a patient, the method comprising:
- determining the level of CT-proET-1 in a sample of a bodily fluid of said patient,
- correlating said level of CT-proET-1 with the therapy stratification of syncope, wherein the patients are stratified according to their need of a cardiac pacemaker by determining the level of CT-proET-1, wherein a level above a predetermined threshold level is indicative of patients suffering from orthostatic hypotension who do not need a cardiac pacemaker, and wherein a level below a predetermined threshold level is indicative of patients suffering from cardioinhibitory reflex who are in need of a cardiac pacemaker.

Syncope is defined as a transient loss of consciousness due to transient global cerebral hypoperfusion characterized by rapid onset, short duration, and spontaneous complete recovery (Moya et al. 2009. Heart J. 30:2631-2671).

VVS is defined as a reproduction of syncope associated with a characteristic pattern of pronounced hypotension and/or bradycardia/ asystole.

CSH is defined as a fall in SBP ≥ 50 mm Hg and/or asystole > 3 sec.

OH is defined as a sustained decrease in systolic BP (SBP) ≥ 20 mm Hg and/or decrease in diastolic BP (DBP) ≥ 10 mm Hg, or systolic BP < 90 mmHg (classical OH, within 3min of tilt phase; and delayed OH, between 3 and 20 min), while POTS has characteristic symptoms of orthostatic intolerance with heart rate increase > 30/min or tachycardia >120/min in standing position. Initial OH is present when a transient BP decrease within 15 s after standing, >40 mmHg SBP and/or >20 mmHg DBP with symptoms of cerebral hypoperfusion occurred and a typical syncope scenario was reported by the patient (Wieling et al. 2007. Clin Sci (Lond). 112:157-165).

Cardioinhibitory syncope is defined as a heart rate fall to a ventricular rate less than 40 bpm for more than 10 sec, or asystole > 3 sec, whereas vasodepressor reflex is defined as a heart rate drop≤10% from its peak at the time of syncope (Brignole et al. 2000. Europace 2:66-76).

As mentioned herein, an "assay" or "diagnostic assay" can be of any type applied in the field of diagnostics. Such an assay may be based on the binding of an analyte to be detected to one or more capture probes with a certain affinity. Concerning the interaction between capture molecules and target molecules or molecules of interest, the affinity constant is preferably greater than 10⁸ M⁻¹.

In the context of the present invention, "capture molecules" are molecules which may be used to bind target molecules or molecules of interest, i.e. analytes (i.e. in the context of the present invention the peptide(s)), from a sample. Capture molecules must thus be shaped adequately, both spatially and in terms of surface features, such as surface charge, hydrophobicity, hydrophilicity, presence or absence of Lewis donors and/or acceptors, to specifically bind the target molecules or molecules of interest. Hereby, the binding may for instance be mediated by ionic, van-der-Waals, pi-pi, sigma-pi, hydrophobic or hydrogen bond interactions or a combination of two or more of the aforementioned interactions between the capture molecules and the target molecules or molecules of interest. In the context of the present invention, capture molecules may for instance be selected from the group comprising a nucleic acid molecule, a carbohydrate molecule, a PNA molecule, a protein, an antibody, a peptide or a glycoprotein. Preferably, the capture molecules are antibodies, including fragments thereof with sufficient affinity to a target or molecule of interest, and including recombinant antibodies or recombinant antibody fragments, as well as chemically and/or biochemically modified derivatives of said antibodies or fragments derived from the variant chain with a length of at least 12 amino acids thereof.

The preferred detection methods comprise immunoassays in various formats such as for instance radioimmunoassay (RIA), chemiluminescence- and fluorescence-immunoassays, Enzyme-linked immunoassays (ELISA), Luminex-based bead arrays, protein microarray assays, and rapid test formats such as for instance immunochromatographic strip tests.

The assays can be homogenous or heterogeneous assays, competitive and non-competitive assays. In a particularly preferred embodiment, the assay is in the form of a sandwich assay, which is a non-competitive immunoassay, wherein the molecule to be detected and/or quantified is bound to a first antibody and to a second antibody. The first antibody may be bound to a solid phase, e.g. a bead, a surface of a well or other container, a chip or a strip, and the second antibody is an antibody which is labeled, e.g. with a dye, with a radioisotope, or a reactive or catalytically active moiety. The amount of labeled antibody bound to the analyte is then measured by an appropriate method. The general composition and procedures involved with "sandwich assays" are well-established and known to the skilled person (The Immunoassay Handbook, Ed. David Wild, Elsevier LTD, Oxford; 3rd ed. (May 2005), ISBN-13: 978-0080445267*;* Hultschig C et al., Curr Opin Chem Biol. 2006 Feb; 10(1):4-10. PMID: 16376134*,* incorporated herein by reference).

In a particularly preferred embodiment the assay comprises two capture molecules, preferably antibodies which are both present as dispersions in a liquid reaction mixture, wherein a first labeling component is attached to the first capture molecule, wherein said first labeling component is part of a labeling system based on fluorescence- or chemiluminescence-quenching or amplification, and a second labeling component of said marking system is attached to the second capture molecule, so that upon binding of both capture molecules to the analyte a measurable signal is generated that allows for the detection of the formed sandwich complexes in the solution comprising the sample.

Even more preferred, said labeling system comprises rare earth cryptates or rare earth chelates in combination with fluorescence dye or chemiluminescence dye, in particular a dye of the cyanine type.

Even more preferred, said labeling system comprises rare earth cryptates or rare earth chelates in combination with a fluorescence dye or chemiluminescence dye, in particular a dye of the cyanine type. In the context of the present invention, fluorescence based assays comprise the use of dyes, which may for instance be selected from the group comprising FAM (5-or 6-carboxyfluorescein), VIC, NED, Fluorescein, Fluoresceinisothiocyanate (FITC), IRD-700/800, Cyanine dyes, such as CY3, CY5, CY3.5, CY5.5, CY7, Xanthen, 6-Carboxy-2',4',7',4,7-hexachlorofluorescein (HEX), TET, 6-Carboxy-4',5'-dichloro-2',7'-dimethoxyfluorescein (JOE), N,N,N',N'-Tetramethyl-6-carboxyrhodamine (TAMRA), 6-Carboxy-X-rhodamine (ROX), 5-Carboxyrhodamine-6G (R6G5), 6-Carboxyrhodamine-6G (RG6), Rhodamine, Rhodamine Green, Rhodamine Red, Rhodamine 110, BODIPY dyes, such as BODIPY TMR, Oregon Green, Coumarines such as Umbelliferone, Benzimides, such as Hoechst 33258; Phenanthridines, such as Texas Red, Yakima Yellow, Alexa Fluor, PET, Ethidiumbromide, Acridinium dyes, Carbazol dyes, Phenoxazine dyes, Porphyrine dyes, Polymethin dyes, and the like.

In the context of the present invention, chemiluminescence based assays comprise the use of dyes, based on the physical principles described for chemiluminescent materials in Kirk-Othmer, Encyclopedia of chemical technology, 4th ed., executive editor, J. I. Kroschwitz; editor, M. Howe-Grant, John Wiley & Sons, 1993, vol.15, p. 518-562*,* incorporated herein by reference, including citations on pages 551-562. Preferred chemiluminescent dyes are acridiniumesters.

"Diagnosis" in the context of the present invention relates to the recognition and (early) detection of a disease or clinical condition in a subject and may also comprise differential diagnosis.

The term "assessment" relates to the evaluation of the severity of the disease in a patient.

In the present invention, the term "prognosis" denotes a prediction of how a subject's (e.g. a patient's) medical condition will progress. This may include an estimation of the chance of recovery or the chance of an adverse outcome for said subject (e.g. recurrence of a syncopal attack or death).

The term "patient" as used herein refers to a living human or non-human organism that is receiving medical care or that should receive medical care due to a disease. This includes persons with no defined illness who are being investigated for signs of pathology. Thus the methods and assays described herein are applicable to both, human and veterinary disease.

The term "sample" as used herein refers to a sample of bodily fluid obtained for the purpose of diagnosis, prognosis, or evaluation of a subject of interest, such as a patient. Preferred test samples include blood, serum, plasma, cerebrospinal fluid, urine, saliva, sputum, and pleural effusions. In addition, one of skill in the art would realize that some test samples would be more readily analyzed following a fractionation or purification procedure, for example, separation of whole blood into serum or plasma components.

Thus, in a preferred embodiment of the invention the sample is selected from the group comprising a blood sample, a serum sample, a plasma sample, a cerebrospinal fluid sample, a saliva sample and a urine sample or an extract of any of the aforementioned samples. Preferably, the sample is a blood sample, most preferably a serum sample or a plasma sample.

The term "correlating", as used herein in reference to the use of diagnostic and prognostic marker(s), refers to comparing the presence or level of the marker(s) in a patient to its presence or amount in persons known to suffer from, or known to be at risk of, a given condition. A marker level in a patient sample can be compared to a level known to be associated with a specific diagnosis. The sample's marker level is said to have been correlated with a diagnosis; that is, the skilled artisan can use the marker level to determine whether the patient suffers from a specific type of a disease, and respond accordingly. Alternatively, the sample's marker level can be compared to a marker level known to be associated with a good outcome (e.g. the absence of disease etc.). In preferred embodiments, a panel of marker levels is correlated to a global probability or a particular outcome.

The sensitivity and specificity of a diagnostic and/or prognostic test depends on more than just the analytical "quality" of the test, they also depend on the definition of what constitutes an abnormal result. In practice, Receiver Operating Characteristic curves (ROC curves), are typically calculated by plotting the value of a variable versus its relative frequency in "normal" (i.e. apparently healthy) and "disease" populations (i.e. patients suffering from diabetes, insulin resistance and/or metabolic syndrome). For any particular marker, a distribution of marker levels for subjects with and without a disease will likely overlap. Under such conditions, a test does not absolutely distinguish normal from disease with 100% accuracy, and the area of overlap indicates where the test cannot distinguish normal from disease. A threshold is selected, above which (or below which, depending on how a marker changes with the disease) the test is considered to be abnormal and below which the test is considered to be normal. The area under the ROC curve is a measure of the probability that the perceived measurement will allow correct identification of a condition. ROC curves can be used even when test results do not necessarily give an accurate number. As long as one can rank results, one can create a ROC curve. For example, results of a test on "disease" samples might be ranked according to degree (e.g. 1=low, 2=normal, and 3=high). This ranking can be correlated to results in the "normal" population, and a ROC curve created. These methods are well known in the art (See, *e.g.,* Hanley et al. 1982. Radiology 143: 29-36). Preferably, ROC curves result in an AUC of greater than about 0.5, more preferably greater than about 0.7, still more preferably greater than about 0.8, even more preferably greater than about 0.85, and most preferably greater than about 0.9. The term "about" in this context refers to +/- 5% of a given measurement.

The horizontal axis of the ROC curve represents (1-specificity), which increases with the rate of false positives. The vertical axis of the curve represents sensitivity, which increases with the rate of true positives. Thus, for a particular cut-off selected, the value of (1-specificity) may be determined, and a corresponding sensitivity may be obtained. The area under the ROC curve is a measure of the probability that the measured marker level will allow correct identification of a disease or condition. Thus, the area under the ROC curve can be used to determine the effectiveness of the test.

In certain embodiments, markers and/or marker panels are selected to exhibit at least about 70% sensitivity, more preferably at least about 80% sensitivity, even more preferably at least about 85% sensitivity, still more preferably at least about 90% sensitivity, and most preferably at least about 95% sensitivity, combined with at least about 70% specificity, more preferably at least about 80% specificity, even more preferably at least about 85% specificity, still more preferably at least about 90% specificity, and most preferably at least about 95% specificity. In particularly preferred embodiments, both the sensitivity and specificity are at least about 75%, more preferably at least about 80%, even more preferably at least about 85%, still more preferably at least about 90%, and most preferably at least about 95%. The term "about" in this context refers to +/- 5% of a given measurement.

Threshold levels can be obtained for instance from a Kaplan-Meier analysis, where the occurrence of a disease is correlated with the quartiles of the cardiovascular markers in the population. According to this analysis, subjects with cardiovascular marker levels above the 75th percentile have a significantly increased risk for getting the diseases according to the invention. This result is further supported by Cox regression analysis with full adjustment for classical risk factors: The highest quartile versus all other subjects is highly significantly associated with increased risk for getting a disease according to the invention.

Other preferred cut-off values are for instance the 90th, 95th or 99th percentile of a normal population. By using a higher percentile than the 75th percentile, one reduces the number of false positive subjects identified, but one might miss to identify subjects, who are at moderate, albeit still increased risk. Thus, one might adopt the cut-off value depending on whether it is considered more appropriate to identify most of the subjects at risk at the expense of also identifying "false positives", or whether it is considered more appropriate to identify mainly the subjects at high risk at the expense of missing several subjects at moderate risk.

Other mathematical possibilities to calculate an individual's risk by using the individual's cardiovascular marker level value and other prognostic laboratory and clinical parameters are for instance the NRI (Net Reclassification Index) or the IDI (Integrated Discrimination Index). The indices can be calculated according to Pencina (Pencina MJ, et al.: Evaluating the added predictive ability of a new marker: from area under the ROC curve to reclassification and beyond. Stat Med. 2008; 27:157-172).

The term "level" in the context of the present invention relates to the concentration (preferably expressed as weight/volume; w/v) of marker peptides taken from a sample of a patient.

As mentioned herein in the context of proteins and other peptides, the term "fragment" refers to smaller proteins or peptides derivable from larger proteins or peptides, which hence comprise a partial sequence of the larger protein or peptide. Said fragments are derivable from the larger proteins or peptides by saponification of one or more of its peptide bonds. "Fragments" of the markers PCT, proANP, proBNP, proET-1, proADM and proAVP preferably relate to fragments of at least 6 amino acids in length, most preferably at least 12 amino acid residues in length. Such fragments are preferably detectable with immunological assays as described herein.

Pro-natriuretic peptides are selected from the group consisting of pro-atrial natriuretic peptide (proANP) and pro-brain natriuretic peptide (proBNP) or fragments thereof of at least 12 amino acids in length.

The sequence of the 153 amino acid pre-proANP is shown in SEQ ID NO:1. Upon cleavage of an N-terminal signal peptide (25 amino acids) and the two C-terminal amino acids, proANP (SEQ ID NO:2) is released. This prohormone is cleaved into the mature 28 amino acid peptide ANP, also known as ANP (1-28) or α-ANP, and the amino terminal proANP fragment (1-98) (NT-proANP, SEQ ID NO:3). Mid-regional proANP (MR-proANP) is defined as NT-proANP or any fragments thereof comprising at least amino acid residues 53-90 (SEQ ID NO:4) of proANP.

The amino acid sequence of the precursor peptide of Adrenomedullin (pre-pro-Adrenomedullin) is given in SEQ ID NO:5. Pro-Adrenomedullin relates to amino acid residues 22 to 185 of the sequence of pre-pro-Adrenomedullin. The amino acid sequence of pro-Adrenomedullin (proADM) is given in SEQ ID NO:6. MR-pro-Adrenomedullin (MR-proADM) relates to amino acid residues 45-92 of pre-proADM. The amino acid sequence of MR-proADM is provided in SEQ ID NO:7.

The sequence of the 164 amino acid precursor peptide of Vasopressin (pre-pro-Vasopressin) is given in SEQ ID NO:8. Pro-Vasopressin relates to the amino acid residues 19 to 164 of the sequence of pre-pro-Vasopressin. The amino acid sequence of pro-Vasopressin is given in SEQ ID NO:9. Pro-Vasopressin is cleaved into mature Vasopressin, Neurophysin II and C-terminal proVasopressin (CT-proAVP or Copeptin). Copeptin relates to amino acid residues 126 to 164 of pre-pro-Vasopressin. The amino acid sequence of Copeptin is provided in SEQ ID NO:10. Neurophysin II comprises the amino acid residues 32 to 124 of pre-pro-Vasopressin and its sequence is shown in SEQ ID NO:11.

Procalcitonin is a precursor of calcitonin and katacalcin. The amino acid sequence of PCT 1-116 is given in SEQ ID NO:12.

The sequence of the 134 amino acid precursor peptide of brain natriuretic peptide (pre-pro-BNP) is given in SEQ ID NO:13. Pro-BNP relates to amino acid residues 27 to 134 of pre-pro-BNP. The sequence of pro-BNP is shown in SEQ ID NO:14. Pro-BNP is cleaved into N-terminal pro-BNP (NT-pro-BNP) and mature BNP. NT-pro-BNP comprises the amino acid residues 27 to 102 and its sequence is shown in SEQ ID NO:15. The SEQ ID NO:16 shows the sequence of BNP comprising the amino acid residues 103 to 134 of the pre-pro-BNP peptide.

The sequence of the 212 amino acid precursor peptide of Endothelin-1 (pre-pro-Endothelin-1) is given in SEQ ID NO:17. Pro-ET-1 relates to the amino acid residues 18 to 212 of the sequence of pre-pro-ET-1. The amino acid sequence of pro-ET-1 is given in SEQ ID NO:18. Pro-ET-1 is cleaved into mature ET-1, big-ET-1 and C-terminal proET-1 (CT-proET-1). ET-1 relates to the amino acid residues 53 to 73 of pre-pro-ET-1. The amino acid sequence of ET-1 is shown in SEQ ID NO:19. CT-proET-1 relates to amino acid residues 168 to 212 of pre-pro-ET-1. The amino acid sequence of CT-proET-1 is provided in SEQ ID NO:20. Big-ET-1 comprises the amino acid residues 53 to 90 of pre-pro-ET-1 and its sequence is shown in SEQ ID NO:21.

The patient is diagnosed with having orthostatic hypotension (all) when said determined CT-proET-1 level at baseline is higher than a predetermined threshold level. Preferably, the predetermined threshold level is between 30 and 70 pmol/L, more preferably between 45 and 70 pmol/L, even more preferred between 55 and 70 pmol/L, most preferred between 60 and (above) 70 pmol/L. The patient is diagnosed with having a OH when said determined CT-proET-1 level at baseline is higher than 30 pmol/L, preferably higher than 40 pmol/L, more preferably higher than 45 pmol/L, even more preferably higher than 55 pmol/L, even more preferably higher than 60 pmol/L, most preferred higher than 70 pmol/L.

It is even more preferred that the patient is diagnosed with not having orthostatic hypotension (all) when said determined CT-proET-1 level at baseline is lower than a predetermined threshold level. Preferably, the predetermined threshold level is between 30 and 70 pmol/L, more preferably between 30 and 60 pmol/L, even more preferred between 30 and 55 pmol/L, most preferred between 30 and 45 pmol/L. It is even more preferred that the patient is diagnosed with not having OH when said determined CT-proET-1 level at baseline is lower than 70 pmol/L, preferably lower than 60 pmol/L, more preferably lower than 55 pmol/L, even more preferably lower than 45 pmol/L, most preferred lower than 30 pmol/L.

The patient is diagnosed with having CSH when said determined MR-proANP level at baseline is lower than a predetermined threshold level. Preferably, the predetermined threshold level is between 50 and 200 pmol/L, more preferably between 50 and 150 pmol/L, even more preferred between 50 and 100 pmol/L, most preferred between 50 and 85 pmol/L. It is preferred that the patient is diagnosed with having CSH when said determined MR-proANP level at baseline is lower than 200 pmol/L, preferably lower than 150 pmol/L, more preferably lower than 100 pmol/L, even more preferably lower than 85 pmol/L, most preferred lower than 50 pmol/L.

It is even more preferred that the patient is diagnosed with not having CSH when said determined MR-proANP level at baseline is higher than a predetermined threshold level. Preferably, the predetermined threshold level is between 50 and 200 pmol/L, more preferably between 85 and 200 pmol/L, even more preferred between 100 and 200 pmol/L, most preferred between 150 and 200 pmol/L. It is even more preferred that the patient is diagnosed with not having CSH when said determined MR-proANP level at baseline is higher than 50 pmol/L, preferably higher than 85 pmol/L, more preferably higher than 100 pmol/L, even more preferably higher than 150 pmol/L, most preferred higher than 200 pmol/L.

The patient is diagnosed with having cardioinhibitory reflex when said determined CT-proET-1 level at baseline is lower than a predetermined threshold level. Preferably, the predetermined threshold level is between 30 and 70 pmol/L, more preferably between 30 and 60 pmol/L, even more preferred between 30 and 55 pmol/L, most preferred between 30 and 45 pmol/L. It is preferred that the patient is diagnosed with having cardioinhibitory reflex when said determined CT-proET-1 level at baseline is lower than 70 pmol/L, preferably lower than 60 pmol/L, more preferably lower than 55 pmol/L, even more preferably lower than 45 pmol/L, most preferred lower than 30 pmol/L.

It is even more preferred that the patient is diagnosed with not having cardioinhibitory reflex when said determined CT-proET-1 level at baseline is higher than a predetermined threshold level. Preferably, the predetermined threshold level is between 30 and 70 pmol/L, more preferably between 45 and 70 pmol/L, even more preferred between 55 and 70 pmol/L, most preferred between 60 and 70 pmol/L. It is even more preferred that the patient is diagnosed with not having cardioinhibitory reflex when said determined CT-proET-1 level at baseline is higher than 30 pmol/L, preferably higher than 45 pmol/L, more preferably higher than 55 pmol/L, even more preferably higher than 60 pmol/L, most preferred higher than 70 pmol/L.

The patient is diagnosed with having initial OH when said determined CT-proAVP level at baseline is lower than a predetermined threshold level. Preferably, the predetermined threshold level is between 2.5 and 20 pmol/L, more preferably between 2.5 and 10 pmol/L, even more preferred between 2.5 and 8 pmol/L, even more preferred between 2.5 and 6 pmol/L, most preferred between 2.5 and 4 pmol/L. It is preferred that the patient is diagnosed with having initial OH when said determined CT-proAVP level at baseline is lower than 20 pmol/L, preferably lower than 10 pmol/L, more preferably lower than 8 pmol/L, even more preferably lower than 6 pmol/L, more preferably lower than 4 pmol/L, most preferred lower than 2.5 pmol/L.

It is even more preferred that the patient is diagnosed with not having initial OH when said determined CT-proAVP level at baseline is higher than a predetermined threshold level. Preferably, the predetermined threshold level is between 2.5 and 20 pmol/L, more preferably between 4 and 20 pmol/L, even more preferred between 6 and 20 pmol/L, even more preferred between 8 and 20 pmol/L, most preferred between 10 and 20 pmol/L. It is preferred that the patient is diagnosed with not having initial OH when said determined CT-proAVP level at baseline is higher than 2.5 pmol/L, preferably higher than 4 pmol/L, more preferably higher than 6 pmol/L, even more preferably higher than 8 pmol/L, more preferably higher than 10 pmol/L, most preferred higher than 20 pmol/L.

The patient is diagnosed with having vasovagal syncope when said determined MR-proANP level at baseline is lower than a predetermined threshold level. Preferably, the predetermined threshold level is between 50 and 200 pmol/L, more preferably between 50 and 150 pmol/L, even more preferred between 50 and 100 pmol/L, most preferred between 50 and 85 pmol/L. It is preferred that the patient is diagnosed with having a vasovagal syncope when said determined MR-proANP level at baseline is lower than 200 pmol/L, preferably lower than 150 pmol/L, more preferably lower than 100 pmol/L, even more preferably lower than 85 pmol/L, most preferred lower than 50 pmol/L.

It is even more preferred that the patient is diagnosed with not having vasovagal syncope when said determined MR-proANP level at baseline is higher than a predetermined threshold level. Preferably, the predetermined threshold level is between 50 and 200 pmol/L, more preferably between 85 and 200 pmol/L, even more preferred between 100 and 200 pmol/L, most preferred between 150 and 200 pmol/L. In a preferred embodiment the patient is diagnosed with not having a vasovagal syncope when said determined MR-proANP level at baseline is higher than 50 pmol/L, preferably higher than 85 pmol/L, more preferably higher than 100 pmol/L, even more preferably higher than 150 pmol/L, most preferred higher than 250 pmol/L.

The above mentioned cut-off values might be different in other assays, if these have been calibrated differently from the assay systems used in the present invention. Therefore the above mentioned cut-off values shall apply for such differently calibrated assays accordingly, taking into account the differences in calibration. One possibility of quantifying the difference in calibration is a method comparison analysis (correlation) of the assay in question (e.g. a PCT assay) with the respective biomarker assay used in the present invention (e.g. BRAHMS KRYPTOR PCT sensitive) by measuring the respective biomarker (e.g. PCT) in samples using both methods. Another possibility is to determine with the assay in question, given this test has sufficient analytical sensitivity, the median biomarker level of a representative normal population, compare results with the median biomarker levels as described in the literature (e.g. in EP 09011073.5 for PCT - "Procalcitonin for the prognosis of adverse events in the asymptomatic population") and recalculate the calibration based on the difference obtained by this comparison. With the calibration used in the present invention, samples from normal (healthy) subjects have been measured: median (interquartile range) plasma procalcitonin was 0.018 (0.015 - 0.022) ng/ml in men and 0.014 (0.012 - 0.017) ng/ml in women (Abbasi et al. 2010. JCEM 95:E26-E31), median (range) plasma MR-proADM was 0.41 *(0.23 - 0.64) nmol*/*L (*Smith et al. 2009. Clin Chem 55:1593-1595*),* median (interquartile range) copeptin concentration was 4.7 (2.9 - 7.5) pmol/L, with significantly higher concentration in males (6.2 (4.1-9.5) pmol/L) than in females (3.6 (2.4-5.5) pmol/l) (Meijer et al. 2010. Kidney Int 77.29-36), median (interquartile range) MR-proANP concentration was 66 (51 - 86) pmol/L (Melander et al. 2009. JAMA 302:49-57) and mean (range) plasma CT-proET-1 concentration was 44.3 (10.5 - 77.4) pmol/L (Papassotiriou et al. 2006. Clin Chem 52:1144-1151).

The automated sandwich fluorescence assay for the detection of MR-proADM uses a sheep polyclonal antibody directed against a peptide comprising the amino acids 68 to 86 of the human preproADM sequence (SEQ ID NO: 5) and a sheep polyclonal antibody directed against a peptide comprising the amino acids 83 to 94 of the human preproADM sequence (SEQ ID NO: 5) (Caruhel et al. 2009. Clin Biochem 42:725-8).

The automated sandwich fluorescence assay for the detection of CT-proET-1 uses a mouse monoclonal antibody directed against a peptide comprising the amino acids 167 to 183 of the human proET-1 sequence (SEQ ID NO: 18) and a sheep polyclonal antibody directed against a peptide comprising the amino acids 183 to 195 of the human proET-1 sequence (SEQ ID NO: 18) (Caruhel et al. 2008. Clin Chem 54:A119).

In a preferred embodiment of the invention the levels of at least two biomarkers can be combined.

In one embodiment of the invention serial measurements of biomarkers e.g. at baseline (before the start of an orthostatic tolerance test [for example HUT test or active standing test]) and during or after such a test are carried out. In other words, the levels of CT-proET-1 are determined in at least two samples of a bodily fluid of said patient taken at baseline before the start and during or after an orthostatic tolerance test.

In another preferred embodiment of the invention the at least two levels of the CT-proET-1 can be combined in a mathematical algorithm.

An increase of CT-proET-1 from baseline to standing excludes orthostatic hypotension.

In a further preferred embodiment the at least one level of CT-proET-1 is combined with one or more clinical parameters selected from the group consisting of age, gender, body mass index (BMI), systolic blood pressure, diastolic blood pressure, heart rate, glomerular filtration rate (GFR), total cholesterol, HDL-cholesterol, number of syncopal attacks and the time from the first onset of syncopal attacks.

The term "combination" or "combining" is defined as a possible selection of a certain number of parameters and the arrangement of these parameters into specified groups using a mathematical algorithm (e.g. deviation or ratio). For example, a ratio can be calculated between the level of a biomarker in a sample taken from a patient during or after an orthostatic tolerance test and the level of the same biomarker in a sample taken from the patient at baseline (before the start of an orthostatic tolerance test). Moreover, a deviation can be calculated between the level of a biomarker in a sample taken from a patient during or after an orthostatic tolerance test and the level of the same biomarker in a sample taken from the patient at baseline (before the start of an orthostatic tolerance test). It is also encompassed herein, that a ratio between different biomarkers can be calculated. For example, the ratio can be calculated between biomarker levels measured in samples taken from the patient at the same time point (e.g. a ratio between MR-proADM and CT-proET-1 in a sample taken from the patient at baseline [before the start of an orthostatic tolerance test]) or at different time points (e.g. a ratio between MR-proADM in a sample taken from the patient at baseline and CT-proET-1 in a sample taken from the patient during or after an orthostatic tolerance test).

In another preferred embodiment the level of CT-proET-1 can be combined as continuous or categorical variable.

In a preferred embodiment the levels of the biomarkers MR-proADM and CT-proET-1 are combined.

In another preferred embodiment the levels of the biomarkers CT-proET-1 and MR-proANP are combined.

In another preferred embodiment the levels of the biomarkers CT-proET-1 and Copeptin are combined.

In yet another preferred embodiment the levels of the biomarkers MR-proADM, CT-proET-1 and MR-proANP are combined.

In yet another preferred embodiment the levels of the biomarkers MR-proADM, CT-proET-1 and Copeptin are combined.

In yet another preferred embodiment the levels of the biomarkers CT-proET-1, MR-proANP and Copeptin are combined.

In yet another preferred embodiment the levels of the biomarkers MR-proADM, CT-proET-1, MR-proANP and Copeptin are combined.

The term "score" in the context of the present invention refers to a rating, expressed numerically, based on the specific achievement or the degree to which certain qualities or conditions (*e*.*g*. the level of biomarkers) are present in said patient.

The term "therapy stratification" in the context of the present invention refers to the choice and/or adjustment of a therapeutic treatment of said patient. In the method of the invention, the patients are stratified according to their need of a cardiac pacemaker. The patients are stratified according to their need of a cardiac pacemaker by determining at least the level of CT-proET-1, wherein a level above a predetermined threshold level is indicative of patients suffering from orthostatic hypotension who do not need a cardiac pacemaker, and wherein a level below a predetermined threshold level is indicative of patients suffering from cardioinhibitory reflex who are in need of a cardiac pacemaker.

Cardiac pacing is carried out with a cardiac pacemaker which is a medical device that uses electrical impulses, delivered by electrodes contacting the heart muscles, to regulate the beating of the heart. The primary purpose of a pacemaker is to maintain an adequate heart rate, either because of the heart's native pacemaker is not fast enough, or there is a block in the heart's electrical conduction system. Modern pacemakers are externally programmable and allow the cardiologist to select the optimum pacing modes for individual patients. Cardiac pacing can be performed temporarily or permanently. Permanent pacing with an implantable pacemaker involves transvenous placement of one or more pacing electrodes within a chamber, or chambers, of the heart. There are three basic types of permanent pacemakers, classified according to the number of chambers involved and their basic operating mechanism: single-chamber, dual-chamber and rate-responsive pacemaker.

### Examples

### Study setting, examination protocol and diagnostic criteria

Between September 2008 and April 2011 a total of 255 patients with suspected syncopal episode of unknown etiology (119 men, mean age 60 ± 21 yrs, range 15 to 93) were investigated at the Syncope Unit of Skåne University Hospital in Malmö, a regional tertiary medical center. The first 101 patients were recruited directly from the Emergency Department during an introductory phase as described previously (Fedorowski et al. 2010. Europace 12:1322-1328). When the Syncope Unit was formally established the recruitment of patients was ascertained through referrals from primary care physicians, emergency departments and outpatients or hospital clinics in the Malmö region. Usually, a set of additional examinations, such as Holter-ECG, brain imaging, echocardiography, and exercise - ECG or in some cases EEG, had been performed prior to investigation. As results of these examinations were negative or inconclusive patients were further referred to the Syncope Unit. Patients with clinical signs and test results confirming cardiac (i.e. arrhythmia or structural heart and vessel disease), neurological, metabolic, toxic, and other definite etiology of TLOC were *a priori* excluded, as were those with advanced dementia and physical disability. Study participants were asked to take their regular medication and fast for two hours before the test, although they were allowed to drink water ad libitum. They were also asked to fill in a questionnaire, which explored patients' medical history, including duration and frequency of syncope-related symptoms, as well as current pharmacological treatment. For logistic reasons tests were performed both in the morning (8 to 10 a.m.) and afternoon (13 to 15 p.m.).

Examination was based on an expanded head-up tilt test (HUTT) protocol including peripheral vein cannulation, supine rest of 15 min, blood sampling, carotid sinus massage (CSM) according to Newcastle protocol (Parry et al. 2009. Heart 95:416-420), head-up tilt with repeated blood sampling after 3 min and optional nitroglycerin provocation according to Italian protocol (Bartoletti et al. 2000. Europace 2:339-342)*,* Valsalva maneuver as proposed by Goldstein (Goldstein and Sharabi 2009. Circulation 119:139-146), and finally active standing test according to Wieling (Wieling et al. 2007. Clin Sci (Lond) 112:157-165*).* Blood pressure (BP) and other haemodynamic parameters as well as ECG were continuously traced using a validated Nexfin monitor (BMEYE, The Netherlands) (Eeftinck et al. 2009. Hypertens 22:378-383). In case the haemodynamic response during a specific part of the test was diagnostic we nevertheless proceeded to the next part to investigate a potential overlap between diagnoses. If the results were non-diagnostic or inconclusive the primary study investigator discussed further procedures with cardiac arrhythmia expert who might decide about prolonged ECG monitoring and additional cardiac tests.

The test was diagnostic when a) patient demonstrated typical prodromes (dizziness, lightheadedness, presyncope) and/or reproduced syncope, and b) the haemodynamic responses met the diagnostic criteria of vasovagal reflex (neurally-mediated) syncope (WS, NMS), carotid sinus hypersensitivity (CSH), postural orthostatic tachycardia syndrome (POTS), and orthostatic hypotension (OH) (Moya et al. 2009. Eur Heart J 30:2631-2671*;* Parry et al. 2009. Heart 95:416-420)*.* Briefly, VVS was defined as a reproduction of syncope associated with a characteristic pattern of pronounced hypotension and/or bradycardia/ asystole, CSH as a fall in SBP ≥ 50 mm Hg and/or asystole > 3 sec, OH as a sustained decrease in systolic BP (SBP) ≥ 20 mm Hg and/or decrease in diastolic BP (DBP) ≥ 10 mm Hg, or systolic BP < 90 mmHg (classical OH, within 3 min of tilt phase; and delayed OH, between 3 and 20 min), while POTS has characteristic symptoms of orthostatic intolerance with heart rate increase > 30/min or tachycardia >120/min in standing position. Cardioinhibitory syncope was defined as a heart rate fall to a ventricular rate less than 40 bpm for more than 10 sec, or asystole > 3 sec, whereas vasodepressor reflex was defined as a heart rate drop ≤10% from its peak at the time of syncope (Brignole et al. 2000. Europace 2:66-76)*.* Initial OH was present when a transient BP decrease within 15 s after standing, >40 mmHg SBP and/or >20 mmHg DBP with symptoms of cerebral hypoperfusion occurred and a typical syncope scenario was reported by the patient (Wieling et al. 2007. Clin Sci (Lond). 112:157-165).

If orthostatic challenge during the tilt phase was positive in terms of conventional OH criteria (a persistent SBP fall ≥ 20 mm Hg), but patient did not reproduce symptoms or results were inconclusive, we usually proceeded with nitroglycerine challenge, if standing SBP > 90 mmHg. The nitroglycerine challenge was not diagnostic if patient developed hypotension and tachycardia without the characteristic haemodynamic vasovagal pattern and accompanying symptoms (Moya et al. 2009. Eur Heart J 30:2631-2671)*.* In order to ascertain the accuracy of diagnoses, the digital test records were subsequently inspected off-line using Nexfin@PC (BMEYE, The Netherlands), a software tool developed for visualizing, replaying, and re-analyzing test measurements (incl. ECG) provided by Nexfin system.

### Biomarker Measurement

Blood samples were collected after 15 min rest in supine position before the start of the test. Plasma biomarkers were measured from blood samples (16x250 µl aliquots of EDTA plasma in plastic thermotubes) that had been frozen at -80° C after collection. Copeptin (C-terminal pro-arginine vasopressin; CT-proAVP), a stable peptide of the arginine vasopressin precursor (Fenske et al. 2009. J Clin Endocrinol Metab 94: 123-129), C-terminal endothelin-1 precursor fragment (CT-proET-1) (Papassotiriouet al. 2006. Clin Chem 52:1144-1151)*,* the midregional fragments of pro-atrial natriuretic peptide (MR-proANP) (Morsenthaler et al. 2004. Clin Chem 50:234-236), pro-adrenomedullin (MR-proADM) (Caruhel et al. 2008. Clin Chem 54: A119) and Procalcitonin (Morgenthaler et al. 2002. Clin Chem 48: 788-790) were measured using the following assays according to the instructions of the manufacturer: Thermo Scientific B·R·A·H·M·S CT-proAVP LIA; Thermo Scientific B·R·A·H·M·S CT-proET-1 KRYPTOR; Thermo Scientific B·R·A·H·M·S MR-proANP KRYPTOR; Thermo Scientific B·R·A·H·M·S MR-proADM KRYPTOR; Thermo Scientific B·R·A·H·M·S PCT sensitive LIA (BRAHMS GmbH, Hennigsdorf, Germany).

### Statistical analyses

We applied logistic regression models to assess the relationship between specific diagnoses and the analyzed biomarkers. We performed multivariate adjusted logistic regression analysis, entering VVS, CSH, OH, POTS, initial OH, cardioinhibitory and vasodepressor reflex as the dependent variable, while each of the biomarkers was added as a covariate, respectively. Model 1 was adjusted for age, gender, and BMI, whereas a more comprehensive Model 2 was additionally adjusted for SBP, DBP, heart rate, and estimated GFR. In the next stage, biomarkers significantly associated with a specific diagnosis were entered simultaneously in the Model 2 and we performed backward elimination of covariates to identify independent predictors of specific syncope diagnoses. We then assessed distribution of syncope diagnoses across quartiles of plasma concentrations of the corresponding significantly associated biomarkers. We applied the logistic regression Model 1 and 2 entering quartiles (first or fourth) with the lowest frequency of a specific diagnosis as a reference. Distribution of CSH was limited to those > 60 yrs of age as this state is extremely rare in younger individuals. Distribution of POTS was not assessed as there were only eleven individuals with POTS diagnosis. All calculations were performed using IBM SPSS Statistics software version 19.0 (SPSS Inc., Chicago, IL). All tests were two-sided and a p<0.05 was considered statistically significant.

### Results

As can be seen in **Table 1**, the included patients reported on average seven syncopal attacks over a period of 8 yrs. Moreover, four out of ten patients reported that they were treated for hypertension, mostly with beta-blockers, RAAS inhibitors, and calcium channel blockers. Resting plasma concentrations of the assessed biomarkers are presented in **Table 2**. Based on the results of the expanded HUTT, a total of 142 (56%) patients were diagnosed with VVS, 85 (33%) with OH, 47 (18%) with CSH, and 11 (4%) with POTS. Orthostatic hypotension was equally distributed between the classical (n=42) and delayed form (n=43). In 30 patients (12%) no definite syncope etiology was found but in two of them long-term ECG monitoring demonstrated bradyarrhythmia during syncope and they were subsequently treated with pacemaker. There was an overlap between diagnoses, as OH and POTS frequently precipitated VVS (in 31 and 6 patients, respectively), and 18 patients with VVS fulfilled also CSH criteria. A total of 35 patients (13.7%) had cardioinhibitory syncope during the test; of these 29 were due to vasovagal reflex and 6 due to carotid sinus reflex. In parallel, 32 patients (12.5%) demonstrated a vasodepressor pattern during syncope. Active standing test was performed in 179 patients with history suggestive of initial OH; of these 40 (22.3%) met the diagnostic criteria of initial orthostatic hypotension.

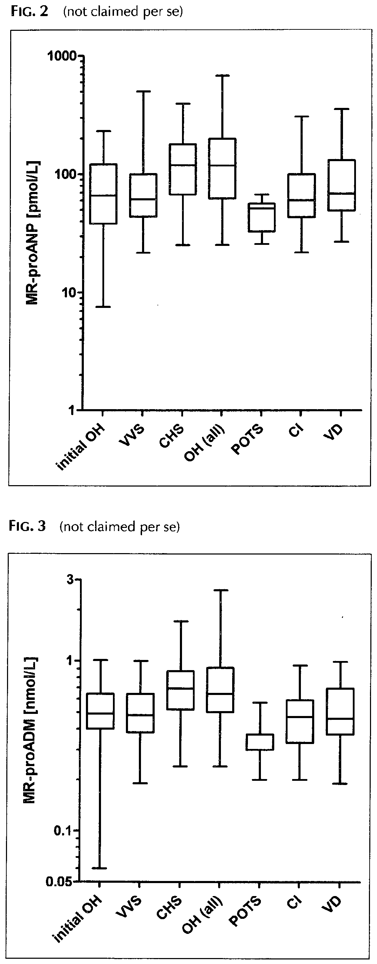

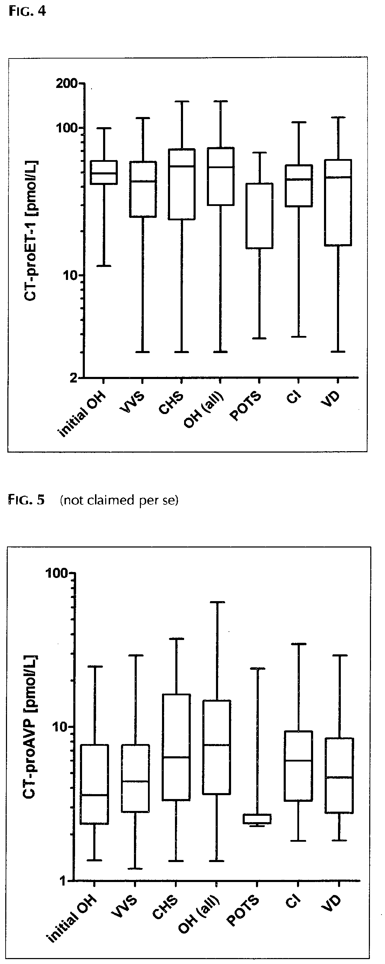

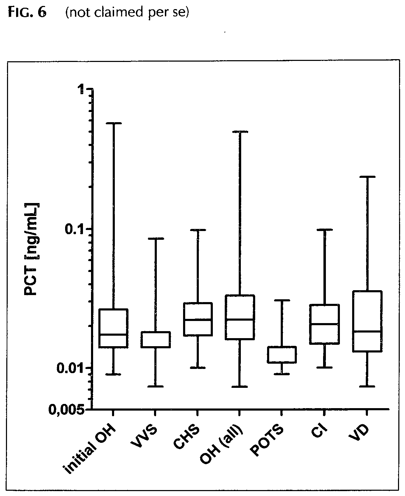

Box-and-whisker plots of single marker values for the different syncope diagnoses are shown in Figures 2 to 6.

In the single-marker multivariate-adjusted model, VVS was significantly associated with lower MR-proANP, MR-proADM, CT-proET-1, CT-proAVP, and procalcitonin, whereas OH with higher renin and CT-proET-1 **(Table 3).** Further, CSH was associated with lower MR-proANP, MR-proADM, and CT-proET-1, as was also cardioinhibitory reflex. A vasodepressor reflex was weakly (p<0.10) correlated with lower procalcitonin and MR-proADM. Finally, POTS was significantly associated with lower procalcitonin, and initial OH with lower CT-proAVP **(Table 3)**. In the multi-marker adjusted Model 2, after backward elimination of covariates, VVS was associated with lower MR-proANP (odds ratio [OR] per one standard deviation [SD]: 0.51, 0.34-0.77; p=0.001), and CT-proET-1 (OR: 0.38, 0.23-0.62; p<0.001), higher resting SBP (OR per 10 mmHg: 1.17, 95% confidence interval: 1.03-1.32, p=0.015), and lower resting heart rate (OR per 10 bpm: 0.76, 0.61-0.95, p=0.020). In parallel, higher CT-proET-1 and renin (OR per one SD: 1.94, 1.27-2.96; p=0.002, and 1.34, 1.01-1.78; p=0.041, respectively), and advancing age (OR per 10 yrs: 1.23, 1.04-1.42, p=0.015) were predictive of OH. Further, CSH was associated with lower MR-proANP (OR per one SD: 0.40, 0.24-0.66; p<0.001) and advancing age (OR per 10 yrs: 2.38, 1.86-2.92; p<0.001), whereas POTS with lower procalcitonin (OR per one SD: 0.21, 0.06-080; p=0.023) and age (OR per 10 yrs: 0.46, 0.44-0.63; p=0.001), female gender (OR: 10.0, 1.1-90.9; p=0.043), and higher resting heart rate (OR per 10 bpm: 1.94, 1.0-2.19; p=0.037). Initial OH was associated with lower CT-proAVP (OR per one SD: 0.57, 0.36-0.89, p=0.013), higher resting DBP (OR per 10 mmHg: 1.11, 1.03-1.18, p=0.003) and, in contrast, lower resting SBP (OR per 10 mmHg: 0.68, 0.56-0.85; p=0.002) and estimated glomerular filtration rate (eGFR) (OR per 10 ml/min: 0.86, 0.78-0.96; p=0.012). Moreover, lower CT-proET-1 was predictive of cardioinhibitory syncope during the test (OR per one SD: 0.34, 0.22-0.53; p<0.001) as was also lower resting heart rate (OR per 10 bpm decrease: 1.45, 1.04-1.89; p=0.037) and male gender (OR: 2.70, 1.17- 6.32; p=0.021). A vasodepressor reflex was inversely correlated with procalcitonin (OR per one SD: 0.59, 0.35-1.00; p= 0.050).

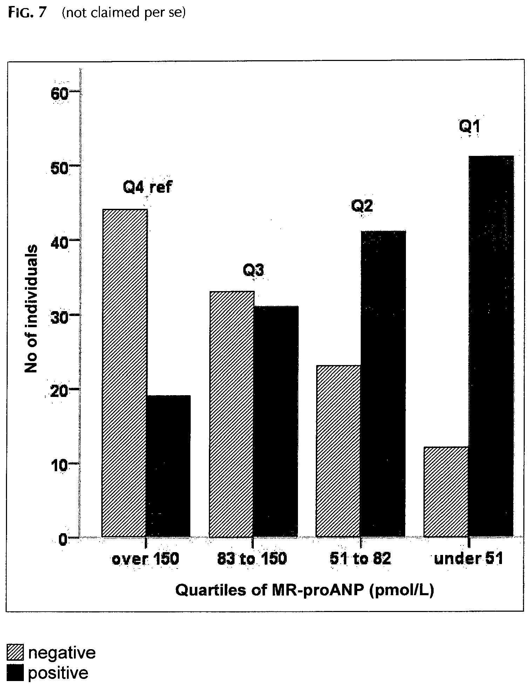

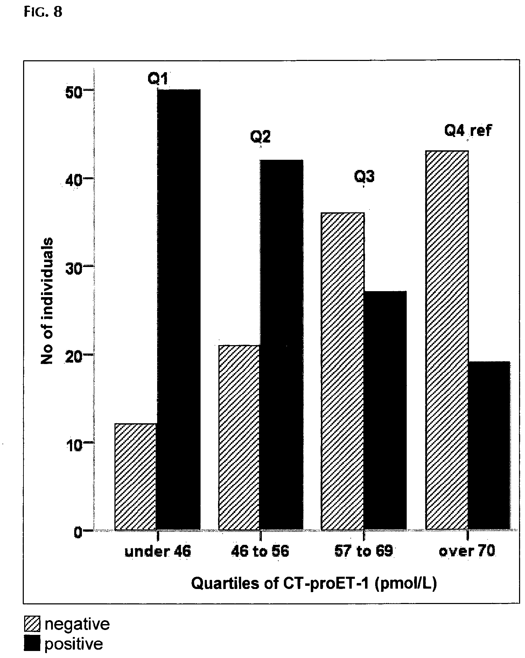

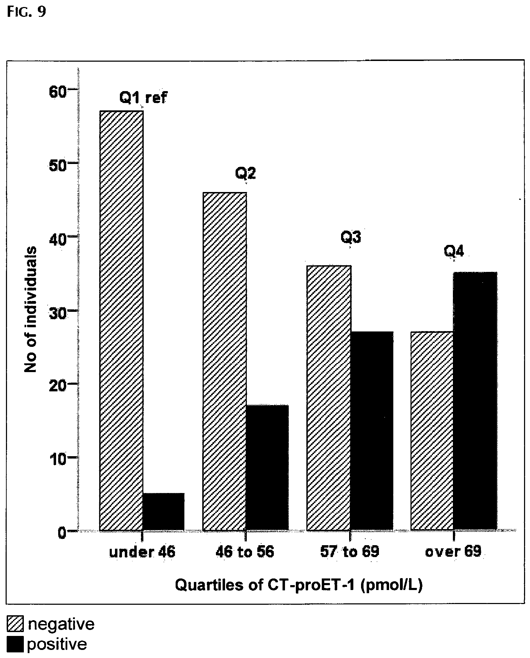

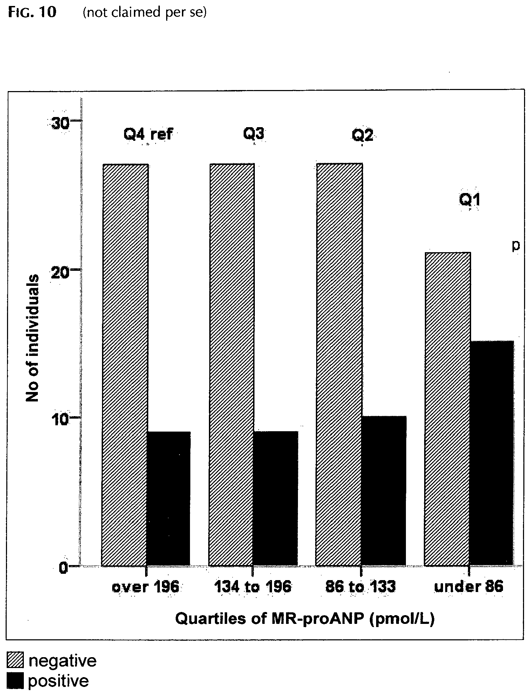

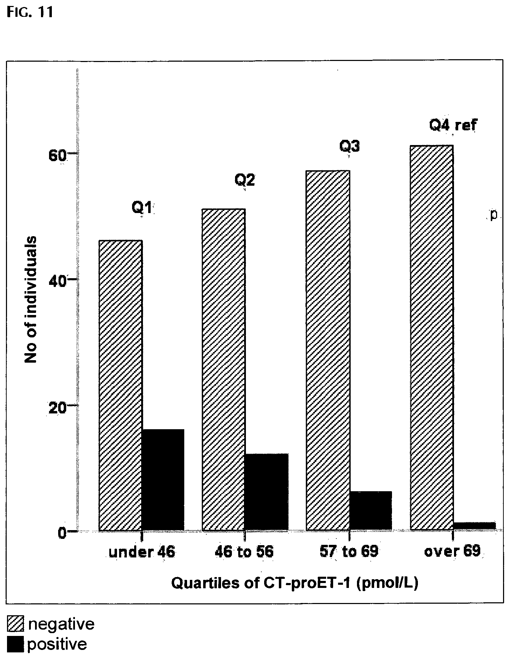

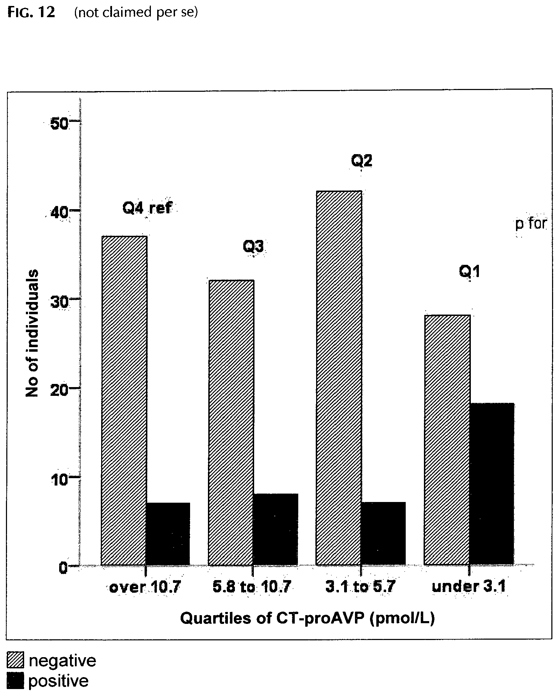

As shown in Figs. 7-12, relations between syncope diagnoses and quartiles of the identified predictive biomarkers followed two distinct patterns: linear and threshold-like. A linear pattern characterized the relationship between MR-proANP and WS (Fig. 7), as well as between CT-proET-1 and VVS (Fig. 8), OH (Fig. 9), and cardioinhibitory reflex (Fig. 11), whereas the threshold effect was observed between CT-proAVP and initial OH (Fig. 12). The relationship between MR-proANP and CSH (Fig. 10) combined both linear and threshold-like effects. The strongest positive predictive value was observed for the lowest quartile of MR-proANP (<51 pmol/L) and CT-proET-1 (<46 pmol/L) in relation to vasovagal reflex, and for the lowest quartile of CT-proAVP (<3.1 pmol/L) in relation to initial OH. In parallel, the lowest quartile of CT-proET-1 was negatively predictive of OH, whereas the highest quartile of CT-proET-1 (>69 pmol/L) generally excluded cardioinhibitory reflex. Summary of logistic regression analyses in the fully adjusted Model 2 for each pair of syncope diagnose and quartiles of the corresponding biomarker is presented in **Table 4**.

The areas under the curve (AUC) of the receiver operating characteristics (ROC) for the single markers and different syncope diagnoses summarized in **Table 5**. Different cut-off values were used to determine the corresponding sensitivity and specificity for each marker to diagnose the respective form of syncope (**Tables 6 to 12**).

For marker combinations logistic regression of the log-transformed biomarker values was performed. A combination of MR-proANP, MR-proADM, CT-proET-1 and Copeptin for the diagnosis of initial OH performed better (OR 0.62 [95% CI 0.41 - 0.91]) than the single markers MR-proANP (OR 0.70 [95% CI 0.48 - 1.00]), MR-proADM (OR 0.72 [95% CI 0.50 - 1.02]), CT-proET-1 (OR 0.70 [95% CI 0.51-0.98]) or Copeptin (OR 0.65 [95% CI 0.44 - 0.96]. Moreover, a combination of MR-proANP, MR-proADM, CT-proET-1 and Copeptin for the diagnosis of vasovagal syncope performed better (OR 0.29 [95% CI 0.20 - 0.42]) than the single markers MR-proANP (OR 0.38 [95% CI 0.28 - 0.52]), MR-proADM (OR 0.35 [95% CI 0.25 - 0.49]), CT-proET-1 (OR 0.36 [95% CI 0.24-0.52]) or Copeptin (OR 0.51 [95% CI 0.38 - 0.68]).

For serial measurements logistic regression was applied using delta log-transformed biomarker values. An increase of Copeptin from baseline to standing showed a strong association with cardioinhibitory reflex (OR 1.48 [95% CI 1.11 - 1.98]). An increase of CT-proET-1 from baseline to standing excluded orthostatic hypotension (OR 0.84 [95% CI 0.65 - 1.08]), whereas an increase of PCT from baseline to standing is significantly associated with POTS (OR 2.42 [95% CI 1.04 - 5.62]).

Moreover, our results show that an increase of Copeptin from baseline to standing was strongly associated with spontaneous VVS (not provoked by nitroglycerin application) (OR 2.54 [95% CI 1.47-4.39]), whereas an increase in MR-proANP from baseline to standing pointed against spontaneous VVS (OR 0.79 [95% CI 0.57 - 1.10)].

### Sequences

SEQ ID NO:1 (amino acid sequence of pre-pro-ANP): 
1       
51      
101     
151     
SEQ ID NO:2 (amino acid sequence of pro-ANP): 
1       NPMYNAVSNA DLMDFKNLLD HLEEKMPLED EVVPPQVLSE PNEEAGAALS
51      PLPEVPPWTG EVSPAQRDGG ALGRGPWDSS DRSALLKSKL RALLTAPRSL
101     RRSSCFGGRM DRIGAQSGLG CNSFRY
SEQ ID NO:3 (amino acid sequence of NT-proANP): 
1      NPMYNAVSNA DLMDFKNLLD HLEEKMPLED EVVPPQVLSE PNEEAGAALS
51     PLPEVPPWTG EVSPAQRDGG ALGRGPWDSS DRSALLKSKL RALLTAPR
SEQ ID NO:4 (amino acid sequence of amino acids 53-90 of proANP):
1 PEVPPWT GEVSPAQRDG GALGRGPWDS SDRSALLKSK L
SEQ ID NO:5 (amino acid sequence of pre-pro-ADM): 
1 MKLVSVALMY LGSLAFLGAD TARLDVASEF RKKWNKWALS RGKRELRMSS
51 SYPTGLADVK AGPAQTLIRP QDMKGASRSP EDSSPDAARI RVKRYRQSMN
101 NFQGLRSFGC RFGTCTVQKL AHQIYQFTDK DKDNVAPRSK ISPQGYGRRR
151 RRSLPEAGPG RTLVSSKPQA HGAPAPPSGS APHFL
SEQ ID NO:6 (amino acid sequence of pro-ADM): 
1 ARLDVASEFR KKWNKWALSR GKRELRMSSS YPTGLADVKA GPAQTLIRPQ
51 DMKGASRSPE DSSPDAARIR VKRYRQSMNN FQGLRSFGCR FGTCTVQKLA
101 HQIYQFTDKD KDNVAPRSKI SPQGYGRRRR RSLPEAGPGR TLVSSKPQAH
151 GAPAPPSGSA PHFL
SEQ ID NO:7 (amino acid sequence of MR-pro-ADM):
1 ELRMSSSYPT GLADVKAGPA QTLIRPQDMK GASRSPEDSS PDAARIRV
SEQ ID NO:8 (amino acid sequence of pre-pro-AVP): 
1 
51 
101 
151 
SEQ ID NO:9 (amino acid sequence of pro-AVP): 
1       CYFQNCPRGG KRAMSDLELR QCLPCGPGGK GRCFGPSICC ADELGCFVGT
51      AEALRCQEEN YLPSPCQSGQ KACGSGGRCA AFGVCCNDES CVTEPECREG
101     FHRRARASDR SNATQLDGPA GALLLRLVQL AGAPEPFEPA QPDAY
SEQ ID NO:10 (amino acid sequence of CT-pre-proAVP or Copeptin):
   1 ASDRSNATQL DGPAGALLLR LVQLAGAPEP FEPAQPDAY
SEQ ID NO:11 (amino acid sequence of Neurophysin II): 
1 AMSDLELRQC LPCGPGGKGR CFGPSICCAD ELGCFVGTAE ALRCQEENYL
51 PSPCQSGQKA CGSGGRCAAF GVCCNDESCV TEPECREGFH RRA
SEQ ID NO:12 (amino acid sequence of PCT): 
1 APFRSALESS PADPATLSED EARLLLAALV QDYVQMKASE LEQEQEREGS
51 SLDSPRSKRC GNLSTCMLGT YTQDFNKFHT FPQTAIGVGA PGKKRDMSSD
101 LERDHRPHVS MPQNAN
SEQ ID NO:13 (amino acid sequence of pre-pro-BNP): 
1       MDPQTAPSRA  LLLLLFLHLA  FLGGRSHPLG  SPGSASDLET  SGLQEQRNHL
51      QGKLSELQVE  QTSLEPLQES  PRPTGVWKSR  EVATEGIRGH  RKMVLYTLRA
101     PRSPKMVQGS  GCFGRKMDRI  SSSSGLGCKV  LRRH
SEQ ID NO:14 (amino acid sequence of pro-BNP): 
1 HPLGSPGSAS DLETSGLQEQ RNHLQGKLSE LQVEQTSLEP LQESPRPTGV
51 WKSREVATEG IRGHRKMVLY TLRAPRSPKM VQGSGCFGRK MDRISSSSGL
101 GCKVLRRH
SEQ ID NO:15 (amino acid sequence of NT-pro-BNP): 
1 HPLGSPGSAS DLETSGLQEQ RNHLQGKLSE LQVEQTSLEP LQESPRPTGV
51 WKSREVATEG IRGHRKMVLY TLRAPR
SEQ ID NO:16 (amino acid sequence of BNP):
   1 SPKMVQGSGC FGRKMDRISS SSGLGCKVLR RH
SEQ ID NO:17 (amino acid sequence of pre-pro-ET-1): 
1       MDYLLMIFSL LFVACQGAPE TAVLGAELSA VGENGGEKPT PSPPWRLRRS
51      KRCSCSSLMD KECVYFCHLD IIWVNTPEHV VPYGLGSPRS KRALENLLPT 101
        KATDRENRCQ CASQKDKKCW NFCQAGKELR AEDIMEKDWN NHKKGKDCSK 151
        LGKKCIYQQL VRGRKIRRSS EEHLRQTRSE TMRNSVKSSF HDPKLKGKPS 201
        RERYVTHNRA HW
SEQ ID NO:18 (amino acid sequence of pro-ET-1): 
1 APETAVLGAE LSAVGENGGE KPTPSPPWRL RRSKRCSCSS LMDKECVYFC
51 HLDIIWVNTP EHVVPYGLGS PRSKRALENL LPTKATDREN RCQCASQKDK
101 KCWNFCQAGK ELRAEDIMEK DWNNHKKGKD CSKLGKKCIY QQLVRGRKIR
151 RSSEEHLRQT RSETMRNSVK SSFHDPKLKG KPSRERYVTH NRAHW
SEQ ID NO:19 (amino acid sequence of ET-1):
   1 CSCSSLMDKE CVYFCHLDII W
SEQ ID NO:20 (amino acid sequence of CT-pro-ET-1):
   1 RSSEEHLRQT RSETMRNSVK SSFHDPKLKG KPSRERYVTH NRAHW
SEQ ID NO:21 (amino acid sequence of Big-ET-1):
   1 CSCSSLMDKE CVYFCHLDII WVNTPEHVVP YGLGSPRS

## Claims

1. A method for the therapy stratification of syncope in a patient, the method comprising:
- determining the level of CT-proET-1 in a sample of a bodily fluid of said patient,
- correlating said level of CT-proET-1 with the therapy stratification of syncope, wherein the patients are stratified according to their need of a cardiac pacemaker by determining the level of CT-proET-1, wherein a level above a predetermined threshold level is indicative of patients suffering from orthostatic hypotension who do not need a cardiac pacemaker, and wherein a level below a predetermined threshold level is indicative of patients suffering from cardioinhibitory reflex who are in need of a cardiac pacemaker.

2. The method according to claim 1, wherein the sample is taken at baseline before the start of an orthostatic tolerance test, preferably the HUT test or active standing test.

3. The method according to claims 1 and 2, wherein at least one sample of a bodily fluid of said patient is taken during or after an orthostatic tolerance test, preferably the HUT test or active standing test.

4. The method according to claims 1 to 3, wherein the levels of CT-proET-1 are determined in at least two samples of a bodily fluid of said patient taken at baseline before the start and during or after an orthostatic tolerance test, preferably the HUT test or active standing test.

5. The method according to claims 1 to 4, wherein the levels of CT-proET-1 are combined in a mathematical algorithm.

6. The method according to any of the preceding claims, wherein the level of CT-proET-1 is combined with at least one clinical parameter selected from the group consisting of age, gender, body mass index (BMI), systolic blood pressure, diastolic blood pressure, heart rate, glomerular filtration rate (GFR), total cholesterol, HDL-cholesterol, age, gender, number of syncopal attacks and the time from the first onset of syncopal attacks.

7. The method according to any of the preceding claims, wherein a differential diagnosis is carried out for at least two of the diseases which are selected from the group consisting of neurally-mediated syncope (NMS), orthostatic hypotension and cardiac syncope.

8. The method according to claim 7, wherein the NMS is selected from the group consisting of WS, CSH, vasodepressor reflex, cardioinhibitory reflex and mixed reflex.

9. The method according to claim 7 or 8, wherein the orthostatic hypotension is selected from the group consisting of initial OH, classical OH, delayed (progressive) OH and POTS.

10. The method according to any one of claims 7 to 9, wherein the cardiac syncope is selected from cardiac syncope due to arrhythmia and cardiac syncope due to a structural disease.

11. The method according to any of the preceding claims, wherein the sample is selected from the group comprising a blood sample, a serum sample, a plasma sample, a cerebrospinal fluid sample, a saliva sample and a urine sample or an extract of any of the aforementioned samples.

12. The method according to any of the preceding claims, wherein the patient is diagnosed with not having orthostatic hypotension when said determined CT-proET-1 level at baseline is lower than a predetermined threshold level.

13. The method according to any of the preceding claims, wherein the patient is diagnosed with not having cardioinhibitory reflex when said determined CT-proET-1 level at baseline is higher than a predetermined threshold level.

14. The method according to any of the preceding claims, wherein CT-proET-1 is detected by a sandwich immunoassay using a first antibody directed against a peptide comprising the amino acids 167 to 183 of the human proET-1 sequence (SEQ ID NO: 18) and a second antibody directed against a peptide comprising the amino acids 183 to 195 of the human proET-1 sequence (SEQ ID NO: 18).

15. The method according to any of the preceding claims, wherein the level of at least one of the biomarkers MR-proANP, MR-proADM and Copeptin is determined in a sample of a bodily fluid of said patient in addition to the level of CT-proET-1.

16. The use of CT-proET-1 for the therapy stratification of syncope, wherein the patients are stratified according to their need of a cardiac pacemaker by determining the level of CT-proET-1, wherein a level above a predetermined threshold level is indicative of patients suffering from orthostatic hypotension who do not need a cardiac pacemaker, and wherein a level below a predetermined threshold level is indicative of patients suffering from cardioinhibitory reflex who are in need of a cardiac pacemaker.

## Patentansprüche

1. Verfahren zur Therapiestratifizierung einer Synkope bei einem Patienten, umfassend:
- Bestimmen des CT-proET-1-Werts in einer Körperflüssigkeitprobe des Patienten,
- Korrelieren des CT-proET-1-Werts mit der Synkopetherapiestratifizierung, wobei die Patienten nach ihrem Bedarf an einem Herzschrittmacher statifiziert werden, indem der CT-proET-1-Wert bestimmt wird, wobei ein Wert oberhalb eines vorbestimmten Schwellenwerts Patienten indiziert, die an einer orthostatischen Hypotension leiden und keinen Herzschrittmacher benötigen, und wobei ein Wert unterhalb eines vorbestimmten Schwellenwerts Patienten indiziert, die an einem kardioinhibitorischen Reflex leiden und einen Herzschrittmacher benötigen.

2. Verfahren nach Anspruch 1, wobei die Probe vor Behandlungsbeginn vor dem Beginn eines orthostatischen Toleranztests entnommen wird, bevorzugt vor dem "HUT-Test" oder "Active-Standing-Test".

3. Verfahren nach Anspruch 1 und 2, wobei mindestens eine Probe einer Körperflüssigkeit des Patienten während eines oder nach einem orthostatischen Toleranztest(s) entnommen wird, vorzugsweise während des oder nach dem HUT-Test(s) oder während des oder nach dem Active-Standing-Test(s).

4. Verfahren nach den Ansprüchen 1 bis 3, wobei der CT-proET-1-Wert in mindestens zwei Körperflüssigkeitsproben des Patienten bestimmt wird, die vor Behandlungsbeginn vor dem Beginn und während eines oder nach einem orthostatischen Toleranztest(s) entnommen werden, bevorzugt während des oder nach dem HUT-Test(s) oder während des oder nach dem Active-Standing-Test(s).

5. Verfahren nach Anspruch 1 bis 4, wobei die CT-proET-1-Werte in einem mathematischen Algorithmus kombiniert werden.

6. Verfahren nach einem der vorhergehenden Ansprüchen, wobei der CT-proET-1-Wert mit mindestens einem klinischen Parameter kombiniert wird, ausgewählt aus der Gruppe, bestehend aus Alter, Geschlecht, Körpermassenindex (BMI), systolischer Blutdruck, diastolischer Blutdruck, Herzfrequenz, glomeruläre Filtrationsrate (GFR), Gesamtcholesterin, HDL-Cholesterin, Alter, Geschlecht, Anzahl synkopaler Anfälle und vergangene Zeit seit dem ersten Auftreten der synkopalen Anfälle.

7. Verfahren nach einem der vorhergehenden Ansprüche, wobei eine Differentialdiagnose für mindestens zwei der Krankheiten durchgeführt wird, die ausgewählt sind aus der Gruppe, bestehend aus neuronal vermittelter Synkope (NMS), orthostatischer Hypotension und kardialer Synkope.

8. Verfahren nach Anspruch 7, wobei die NMS ausgewählt ist aus der Gruppe, bestehend aus VVS, CSH, vasodepressorischer Reflex, kardioinhibitorischer Reflex und gemischter Reflex.

9. Verfahren nach Anspruch 7 oder 8, wobei die orthostatische Hypotension ausgewählt ist aus der Gruppe, bestehend aus anfänglicher OH, klassischer OH, verzögerter (progressiver) OH und POTS.

10. Verfahren nach einem der Ansprüche 7 bis 9, wobei die kardiale Synkope aus kardialer Synkope aufgrund von Arrhythmie und kardialer Synkope aufgrund einer strukturellen Erkrankung ausgewählt ist.

11. Verfahren nach einem der vorhergehenden Ansprüche, wobei die Probe ausgewählt ist aus der Gruppe, bestehend aus einer Blutprobe, einer Serumprobe, einer Plasmaprobe, einer Zerebrospinalflüssigkeitsprobe, einer Speichelprobe und einer Urinprobe oder einem Extrakt aus einer der vorgenannten Proben.

12. Verfahren nach einem der vorhergehenden Ansprüche, wobei der Patient die Diagnose erhält, dass er keine orthostatische Hypotension hat, wenn der bestimmte CT-proET-1-Wert zu Behandlungsbeginn niedriger ist als ein vorbestimmter Schwellenwert.

13. Verfahren nach einem der vorhergehenden Ansprüche, wobei der Patient die Diagnose erhält, dass er keinen kardioinhibitorischen Reflex hat, wenn der bestimmte CT-proET-1-Wert zu Behandlungsbeginn höher ist als ein vorbestimmter Schwellenwert.

14. Verfahren nach einem der vorhergehenden Ansprüche, wobei CT-proET-1 durch einen Sandwich-Immunoassay unter Verwendung eines ersten Antikörpers, der gegen ein Peptid gerichtet ist, das die Aminosäuren 167 bis 183 der humanen proET-1-Sequenz (SEQ ID Nr. 18) umfasst, und eines zweiten Antikörpers, der gegen ein Peptid gerichtet ist, das die Aminosäuren 183-195 der humanen proET-1-Sequenz (SEQ ID Nr. 18) umfasst, ermittelt wird.

15. Verfahren nach einem der vorhergehenden Ansprüche, wobei der Wert von mindestens einem der Biomarker MR-proANP, MR-proADM und Copeptin in einer Körperflüssigkeitsprobe des Patienten zusätzlich zum CT-proET1-Wert bestimmt wird.

16. Die Verwendung von CT-proET-1 für die Therapiestratifizierung von Synkope, wobei die Patienten nach ihrem Bedarf an einem Herzschrittmacher statifiziert werden, indem der CT-proET-1-Wert bestimmt wird, wobei ein Wert oberhalb eines vorbestimmten Schwellenwerts Patienten indiziert, die an einer orthostatischen Hypotension leiden und keinen Herzschrittmacher benötigen, und wobei ein Wert unterhalb eines vorbestimmten Schwellenwerts Patienten indiziert, die an einem kardioinhibitorischen Reflex leiden und einen Herzschrittmacher benötigen.

## Revendications

1. Procédé pour la stratification thérapeutique d'une syncope chez un patient, la méthode comprenant :
- la détermination du taux de CT-proET-1 dans un échantillon d'un fluide corporel dudit patient,
- la corrélation dudit taux de CT-protET-1 avec la stratification thérapeutique de la syncope,
dans lequel les patients sont stratifiés en fonction de leur besoin d'un stimulateur cardiaque par détermination du taux de CT-proET-1, dans lequel un taux supérieur à un seuil prédéterminé est révélateur de patients souffrant d'une hypotension orthostatique qui ne réclame pas de stimulateur cardiaque, et dans lequel un taux inférieur à un seuil prédéterminé est révélateur de patients souffrant d'un réflexe cardio-inhibiteur qui ont besoin d'un stimulateur cardiaque.

2. Procédé selon la revendication 1, dans lequel l'échantillon est prélevé en conditions basales avant le début d'un test de tolérance orthostatique, de manière préférée un test HUT ou un test de station debout active.

3. Procédé selon les revendications 1 et 2, dans lequel au moins un échantillon d'un fluide corporel dudit patient est prélevé pendant ou après un test de tolérance orthostatique, de manière préférée le test HUT ou le test de station debout active.

4. Procédé selon les revendications 1 à 3, dans lequel les taux de CT-proET-1 sont déterminés dans au moins deux échantillons d'un fluide corporel dudit patient, prélevés en conditions basales avant le début et pendant ou après un test de tolérance orthostatique, de manière préférée le test HUT ou le test de station debout active.

5. Procédé selon les revendications 1 à 4, dans lequel les taux de CT-proET-1 sont associés à un algorithme mathématique.

6. Procédé selon l'une quelconque des revendications précédentes, dans lequel les taux de CT-proET-1 sont associés à au moins un paramètre clinique choisi dans le groupe constitué par l'âge, le genre, l'indice de masse corporelle (IMC), la pression artérielle systolique, la pression artérielle diastolique, le rythme cardiaque, le taux de filtration glomérulaire (GFR), le cholestérol total, le cholestérol HDL, l'âge, le genre, le nombre de syncopes et le délai écoulé depuis la première apparition des syncopes.

7. Précédé selon l'une quelconque des revendications précédentes, dans lequel un diagnostic différentiel est effectué pour au moins deux des affections qui sont choisies dans le groupe constitué de la syncope à médiation neuronale (NMS), de l'hypotension orthostatique et de la syncope cardiaque.

8. Procédé selon la revendication 7, dans lequel la NMS est choisie dans le groupe constitué de la syncope vaso-vagale (WS), de la syncope par hypersensibilité sino-carotidienne (CHS), du réflexe vaso-dépresseur, du réflexe cardio-inhibiteur, et du réflexe mixte.

9. Procédé selon la revendication 7 ou 8, dans lequel l'hypotension orthostatique est choisie dans le groupe constitué de l'hypotension orthostatique initiale, de l'hypotension orthostatique classique, de l'hypotension orthostatique retardée (progressive) et du POTS.

10. Procédé selon l'une quelconque des revendications 7 à 9, dans lequel la syncope cardiaque est choisie parmi la syncope cardiaque due à une arythmie et la syncope cardiaque due à une anomalie structurale.

11. Procédé selon l'une quelconque des revendications précédentes, dans lequel l'échantillon est choisi dans le groupe comprenant un échantillon de sang, un échantillon de sérum, un échantillon de plasma, un échantillon de fluide céphalo-rachidien, un échantillon de salive et un échantillon d'urine, ou un extrait de n'importe lequel des échantillons susmentionnés.

12. Procédé selon l'une quelconque des revendications précédentes, dans lequel le patient est diagnostiqué comme n'ayant pas d'hypotension orthostatique lorsque ledit taux de CT-proET-1 déterminé en conditions basales est inférieur à un seuil prédéterminé.

13. Procédé selon l'une quelconque des revendications précédentes, dans lequel le patient est diagnostiqué comme ne présentant pas de réflexe cardio-inhibiteur lorsque ledit taux de CT-proET-1 déterminé en conditions basales est supérieur à un seuil prédéterminé.

14. Procédé selon l'une quelconque des revendications précédentes, dans lequel la CT-proET-1 est détectée par méthode de dosage immunologique en sandwich en utilisant un premier anticorps dirigé contre un peptide comprenant les acides aminés 167 à 183 de la séquence proET-1 humaine (SEQ ID NO : 18) et un second anticorps dirigé contre un peptide comprenant les acides aminés 183 à 195 de la séquence proET-1 humaine (SEQ ID NO : 18).

15. Procédé selon l'une quelconque des revendications précédentes, dans lequel le taux d'au moins l'un des bio-marqueurs MR-proANP, MR-proADM et Co-peptine est déterminé dans un échantillon d'un fluide corporel dudit patient en complément du taux de CT-proET-1.

16. Utilisation de la CT-proET-1 pour la stratification thérapeutique d'une syncope, dans laquelle les patients sont stratifiés en fonction de leur besoin d'un stimulateur cardiaque par détermination du taux de CT-proET-1, dans lequel un taux supérieur à un seuil prédéterminé est révélateur de patients souffrant d'une hypotension orthostatique qui ne réclame pas de stimulateur cardiaque, et dans lequel un taux inférieur à un seuil prédéterminé est révélateur de patients souffrant d'un réflexe cardio-inhibiteur qui ont besoin d'un stimulateur cardiaque.
